# EUROPEAN PATENT APPLICATION

(11) **EP 4 529 869 A1**
(43) Date of publication of application: **02.04.2025**
(21) Application number: 23200614.8
(22) Date of filing: 28.09.2023
(51) Int. Cl.: A61B 17/72, A61B 17/68

(54) **INTRAMEDULLARY NAIL FOR A LONG BONE**

(71) Applicant: Orthofix S.r.l., 37012 Bussolengo (VR) (IT)
(72) Inventor: PRANZETTI, Antony, 37138 Verona (IT); VICENZI, Federico, 37136 Verona (IT); ZACCARIA, Andrea, 37039 Tregnago (VR) (IT)
(74) Representative: Botti & Ferrari S.p.A.

(57) **Abstract**

An intramedullary nail (1) for a long bone, comprising: a tube (2) extending in an axial direction of the intramedullary nail (1), the tube (2) comprising a first through slot (21) longitudinally aligned along the axial direction; a first rod (3) extending in the axial direction and telescopically coupled inside the tube (2), the first rod (3) being further configured for axial displacement within the tube (2). The first rod (3) comprises a first hole (31) transversal to the axial direction and configured for housing a first fixing screw (100), the first hole (31) being configured for axial displacement along the first through slot (21) to displace the first fixing screw (100) in the axial direction. The first rod (3) further comprises: a second hole (32) transversal to the axial direction; a guide element (5) housed in the second hole (32) and axially displaceable in the axial direction, the guide element (5) being enclosed within an outer contour of the tube (2) and configured to prevent a relative rotation of the first rod (3) with respect of the tube (2).

## Description

### Field of application

The present invention relates to the general technical field of orthopedics. More in particular, the invention relates to an intramedullary nail for a long bone.

An intramedullary nail according to the present invention makes it possible to perform bone transport in long bones, in connection with lengthening of long bones or joint fusion, without the use of external fixators.

### Prior art

Intramedullary nails are known from the state of the art, which enable long bones to be distracted in various manners, depending on the specific orthopaedical application.

For example, Two bone fragments can be displaced relative to one another by means of an intramedullary nail including a drive. At the contact point between the two bone fragments, bone is intended to grow anew.

Intramedullary nails are in particular suitable for treating long bones, which include: thigh bones (femur) and shinbones (tibia), upper arm bones (humerus), ell bones (ulna), radius bones (radius) and splinter bones (fibula).

With appropriate preoperative planning, an intramedullary nail makes it possible to provide axial and torsional corrections as part of limb lengthening.

Document WO2021032823 (A1) relates to an intramedullary nail for distracting a long bone comprising a first tube extending in an axial direction of the intramedullary nail, a second tube extending in an axial direction of the intramedullary nail, which is coupled with the first tube for axial displacement within one another, a first locking opening in an end area of the first tube facing away from the second tube, and a coil for energizing a drive.

Unfortunately, known intramedullary nails are not fully effective for treatments under certain conditions.

For example, when there are significant bone losses e.g., longer than 2-3 cm between the proximal and distal bone part, biology would not allow the two bone stumps to come into contact by shortening the limb and then proceeding to distracting the bone callus.

In particular, in known intramedullary nails, the maximum elongation that can be provided to a long bone is limited by the displacement of a fixing screw which is inserted in a hole moving along a slot on the body of the intramedullary nail. Once the fixing screw reaches the end of such slot, no further displacement would be possible.

If the lengthening that an intramedullary nail can provide to a long bone is not sufficient, it becomes necessary to complete surgery by swapping out the previous intramedullary nail and implanting a new intramedullary nail for further lengthening the long bone.

Moreover, it is risky to operate the drive of the intramedullary nail without a respective pin inside the slot, since an internal rod may rotate inside the tubular body and a hole for a fixing screw may become inaccessible.

### Summary of the invention

A general object of the present invention is to overcome drawbacks of the prior art.

A particular object of the present invention is to provide an intramedullary nail for a long bone which is more effective.

A further particular object of the present invention is to provide an intramedullary nail for a long bone which allows to achieve a more effective bone transport, especially for bone lengthening.

A further particular object of the present invention is to provide an intramedullary nail for a long bone which can be better employed to correct a variety of bone defects.

A further particular object of the present invention is to provide an intramedullary nail for a long bone which is more reliable in displacing a rod inside a tubular body, transporting a fixing screw.

These and other objects are achieved by an intramedullary nail for a long bone.

The intramedullary nail comprises a tube extending in an axial direction, the tube comprising a first through slot longitudinally aligned along the axial direction.

The intramedullary nail comprises a first rod extending in the axial direction and telescopically coupled inside the tube; the first rod is configured for axial displacement within the tube.

The first rod comprises a first hole transversal to the axial direction and configured to house a first fixing screw.

The first hole is configured for axial displacement along the first through slot to displace the first fixing screw.

The first rod further comprises a second hole transversal to the axial direction.

The first rod further comprises a guide element housed in the second hole and axially displaceable in the axial direction.

The guide element is enclosed within an outer contour of the tube and is configured to prevent a relative rotation of the first rod with respect of the tube.

Advantageously, the guide element constraints the rotation of the first rod within the tube, so as to maintain the first hole within the profile of the first through slot, even during activation of the drive.

Advantageously, the smaller guide element remains inside the volume defined by the tube of the intramedullary nail.

In particular, advantageously, the guide element does not protrude outside of the body of the tube defined by the outer contour of the first through slot, so that during the axial displacement the guide element does not interfere with the surrounding tissue, improving tolerability of the implant.

Moreover, advantageously, the guide element allows to axially displace the first rod within the tube even if a fixing screw is removed from the first hole. This allows to 'recharge' the distraction displacement provided by the intramedullary nail. Specifically, when a maximum displacement is reached by the intramedullary nail, but a further bone transport is needed for bone lengthening, it is possible to temporarily remove the fixing screw from the first hole and activating the drive to displace the rod in a reverse axial direction. The fixing screw is then inserted in the first hole again, fixing in a new bone section, and transport can continue with the same intramedullary nail in order to achieve the required lengthening.

Advantageously, the guide element allows reverse displacement in axial direction of the rod, without incurring in rotation of the rod within the body that may hinder access to the first hole for the fixing screw.

Moreover, advantageously, the guide element does not protrude outside of the outer contour of the first through slot so that, also during the reverse axial displacement, the guide element does not interfere with the surrounding tissue, avoiding excessive damage to the surroundings during the further surgical review.

Preferably, the guide element comprises a threaded guide screw inserted in the in the second hole, which can be swiftly un-threaded and removed therefrom.

Further features and advantages will be more apparent from the following detailed description of preferred non-limiting embodiments of the present invention, and from the dependent claims which outline preferred and particularly advantageous embodiments of the invention.

### Brief description of the drawings

The invention is illustrated with reference to the following figures, given by way of non-limiting examples, in which:
- Figure 1 shows a top view of a preferred embodiment of intramedullary nail for a long bone according to the invention.
- Figure 2 shows a partial view of the intramedullary nail of Figure 1 in disassembled configuration.
- Figure 3 shows a partial cross-sectional view from the side of the intramedullary nail of Figure 1, in the region of a first through slot thereof, and a disassembled guide element.
- Figure 4 shows a view corresponding to Figure 3 with an assembled guide element.
- Figure 5 shows a perspective view of the intramedullary nail of Figure 1.
- Figure 6 shows a sectional view of the intramedullary nail of Figure 1, with three fixing screws.
- Figure 7 shows a further perspective view of the intramedullary nail of Figure 1, with a fixing screw.
- Figure 8 shows a first application of the intramedullary nail of Figure 1 to a long bone.
- Figure 9 shows a second application of the intramedullary nail of Figure 1 across a knee joint.

In the different figures, analogous elements will be identified with analogous reference numbers.

Moreover, in the figures, if there is a plurality of elements which are similar to each other, only one (or only some) of them will be indicated by a reference number for greater clarity; the other similar elements, although not indicated by a suitable reference number, are to be understood as included by analogy.

### Detailed description

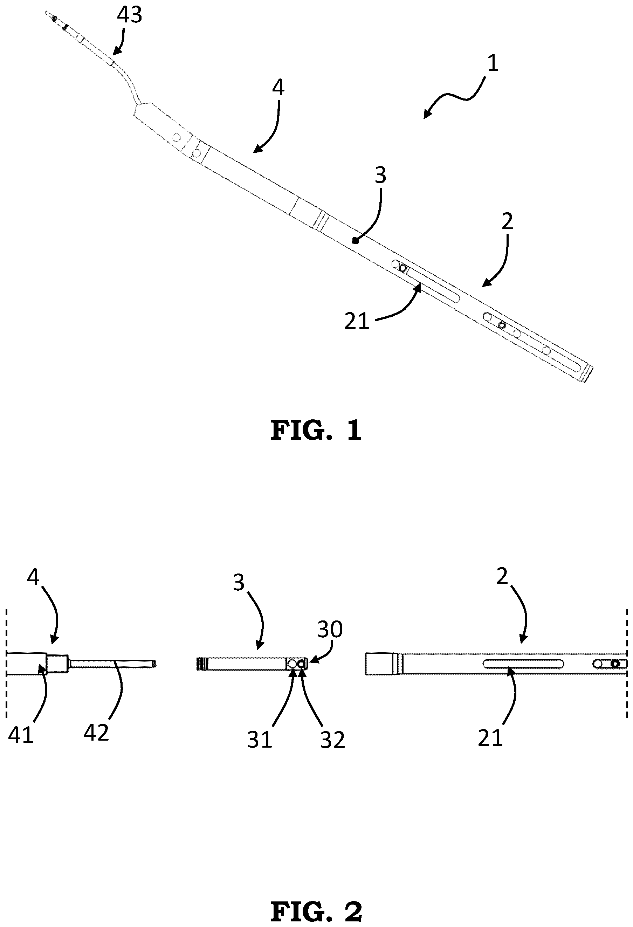

A preferred, and in any case non-limiting, embodiment of intramedullary nail 1 for a long bone according to the invention is shown in Figure 1.

The intramedullary nail 1 comprises, among other elements, a tube 2 extending in an axial direction of the intramedullary nail 1.

The tube 2 comprises a hollow shell, as it will be further described, and comprises a first through slot 21 longitudinally aligned along the axial direction.

The terms "axial" and "radial" herein are to be understood with respect to the longitudinal axis of the intramedullary nail 1, with the longitudinal axis extending in particular along the intramedullary nail 1 or the largest spatial expansion of the intramedullary nail 1. In particular, an axial direction is to be understood as a direction along or in parallel to the longitudinal axis of the intramedullary nail 1, a radial direction is to be understood as a direction perpendicular to the longitudinal axis. The longitudinal axis of an intramedullary nail 1 may also be curved, for example, in an intramedullary nail 1 having a slight bend for lower legs. Typically, the intramedullary nail 1 is at least substantially circular in a cross section to the longitudinal axis of the intramedullary nail 1.

As it will be further described, the intramedullary nail 1 can be coupled with a receiver (not shown) connected by an energy feed line. An external control electronics (not shown) and a transmitter (not shown) can be provided. The energy required by the drive is transmitted to the receiver implanted beneath the skin by applying the transmitter, so that there is no contact between the implanted intramedullary nail 1 and the surface (skin) of the body.

The intramedullary nail 1 makes it possible to perform bone transport in long bones, lengthening, or joint fusion without the use of external fixators. With appropriate preoperative planning, it is possible to make axial and torsional corrections as part of limb lengthening.

Preferably, the intramedullary nail 1 is composed of two or more interconnected elements, which can be assembled, welded together, bonded together, or connected by form closure using a joining process. In possible variants, the tube 2 could also comprise two or more tube pieces.

Typically, the tube 2 is made of metal or a metal alloy, in particular of biocompatible metal or a biocompatible metal alloy.

Preferably, some elements of the intramedullary nail 1 are made of plastics, for example, of epoxy resin, silicone, or thermoplastic resin.

Figure 2 shows a partial view of the intramedullary nail 1 in disassembled configuration.

The intramedullary nail 1 comprises a first rod 3 extending in the same axial direction and telescopically coupled inside the tube 2. As it will be further described, the first rod 3 is configured for axial displacement within the tube 2.

The tube 2 with the first through slot 21 provides mounting to the telescopic rod 3. In particular, the first through slot 21 has the function of axially constraining the displacement of the rod 3 in the presence of a protruding body (for example, a bone fixing screw, not shown).

The first rod 3 comprises a first hole 31 transversal to the axial direction and configured to house a first fixing screw (not shown). The first hole 31, made in the first rod 3, is as well configured for axial displacement along the first through slot 21 to displace the first fixing screw for transporting a bone segment, especially for lengthening the long bone as it will be further described.

The first rod 3 further comprises a second hole 32 transversal to the axial direction.

In a preferred, non-limiting, embodiment, the second hole 32 is parallelly aligned with the first hole 31. Nonetheless, the second hole 32 may be non-parallel, as long as it remains configured to move along a slot oriented in the same axial direction. For example, the second hole 32 could be tilted of 90° and moving along a second different slot which is also oriented at 90°. The purpose of this second hole 32 will be further described.

The intramedullary nail 1 comprises a drive 4 configured for axially displacing the first rod 3 within the tube 2. Preferably, the drive 4 comprises an electric motor 41 and a screw transmission 42. The electric motor 41 is adapted to power the screw transmission 42, which in turn acts on the first rod 3 for a reversible displacement thereof.

In general, once the intramedullary nail 1 is implanted, it is preferably powered by an external controller that transfers electrical impulses through the skin to an antenna implanted under the skin. The energy is then transferred to the electric motor 41 of the intramedullary nail 1 via a power cable 43. The electric motor 41, preferably via a gear reduction system, provides a rotary motion to a worm screw 42 on which the first rod 3 containing the first hole 31 of the transport segment is provided.

Preferably, the intramedullary nail 1 comprises an electric power supply device (not shown) for powering the drive 4 via the power cable 43. Preferably such electric power supply device comprises an induction coil.

Preferably, the intramedullary nail 1 and a primary coil connected to the power cable 43 are set up for transcutaneous data transmission with a further external coil. Preferably, the intramedullary nail 1 comprises a data processing unit for sending data via the coil or for reading data received via the coil.

In possible variants, the drive could comprise a gear transmission, an electronics system and/or a sensor system for controlling and monitoring the drive, an electrical energy storage such as a battery.

In yet other possible variants, the drive may comprise a different kind of transmission/motor system, not necessarily electric, for example involving manual operation.

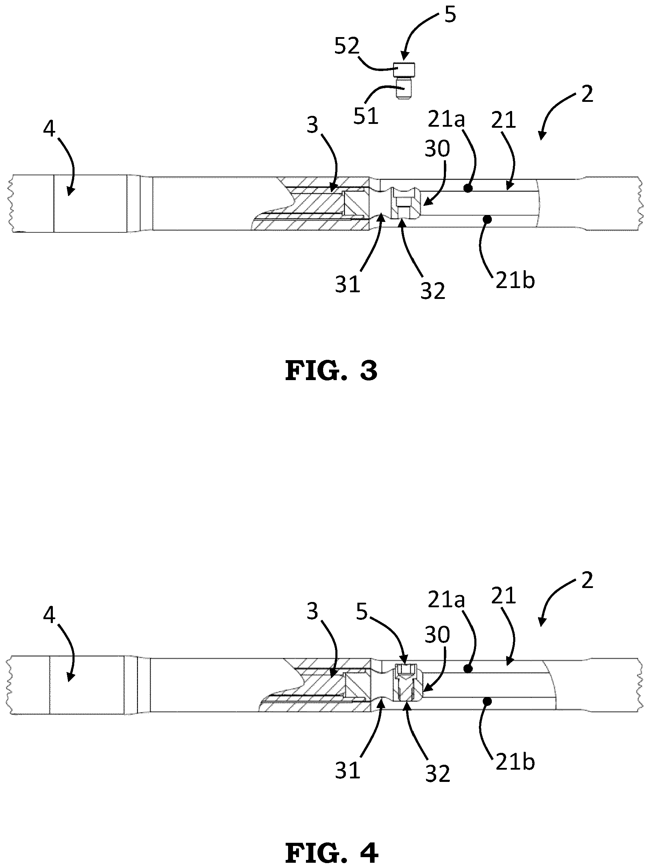

Figure 3 shows a partial cross-sectional view from the side of the intramedullary nail 1, in the region of a first through slot 21 while Figure 4 shows a view corresponding to Figure 3 with an assembled guide element which will be further described.

As it can be seen in the section hatching, the first rod 3 is preferably made of one or more elements assembled together.

The first rod 3 comprises a guide element 5 which can be housed in the second hole 32. In Figure 3 the guide element 5 is separated from the first rod 3, and in Figure 4 the guide element 5 is coupled to the first rod 3 as in the operative condition.

The guide element 5 is axially displaceable, as it is coupled to the axially displaceable first rod 3.

Preferably, the guide element 5 is displaceable along the same first through slot 21 already described. In possible variants, the guide element 5 and its associated second hole 32 could be provided in connection with a dedicated slot, separated from slot 21 but oriented in the same axial direction.

The guide element 5 remains enclosed within an outer contour of the first through slot 21 and is configured to prevent a relative rotation of the first rod 3 with respect of the tube 2.

The second hole 32 may be a through hole or a blind hole.

The second hole 32 which houses the guide element 5 is preferably threaded, and the guide element 5 is preferably a guide screw comprising a threaded stem 51 which is screwed in the second hole 32.

Preferably, the guide screw 5 comprises also a head 52 which remains, during axial displacement, enclosed within the outer contour of the tube 2 so as to not protrude out of the tube 2 itself.

As the guide screw 5 can be unscrewed and removed from the second hole 32, it is possible to remove swiftly the guide element. Thus, if one wants to achieve a little 'extra' displacement, it would be possible to disassemble the guide screw 5 and displace the whole length of the slot 21 up until when the fixing screw 100 enters in contact with the end of the first slot 21, the second hole 32 surpassing the end of the first slot 21 inside of the tube 2.

In possible variants, instead of a screw, the guide element could comprise different elements which can be housed in the second hole 32, for example inserts which are glued or otherwise constrained. Also, the second hole needs not to be circular and could have various shapes, even more complex.

As it can be appreciated from the sectional view, the tube 2 comprises a hollow shell, and the first through slot 21 comprises two respective cutouts 21a and 21b in the hollow shell; the two respective cutouts 21a and 21b are mutually transversally aligned to provide a through slot 21.

In a preferred embodiment, the hollow shell of the tube 2 is cylindrical or substantially cylindrical; in possible variants, the hollow shell may define a polygonal outline.

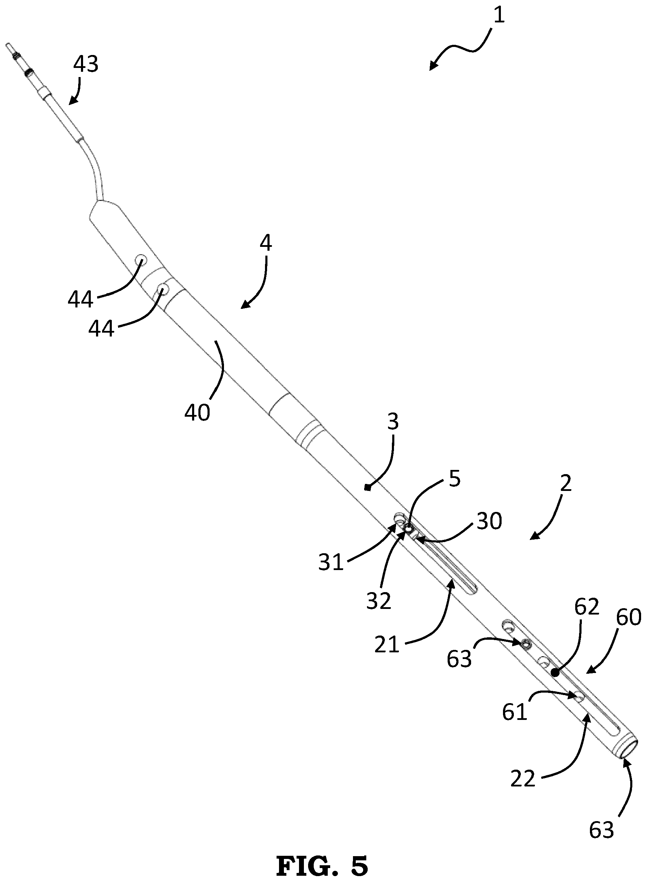

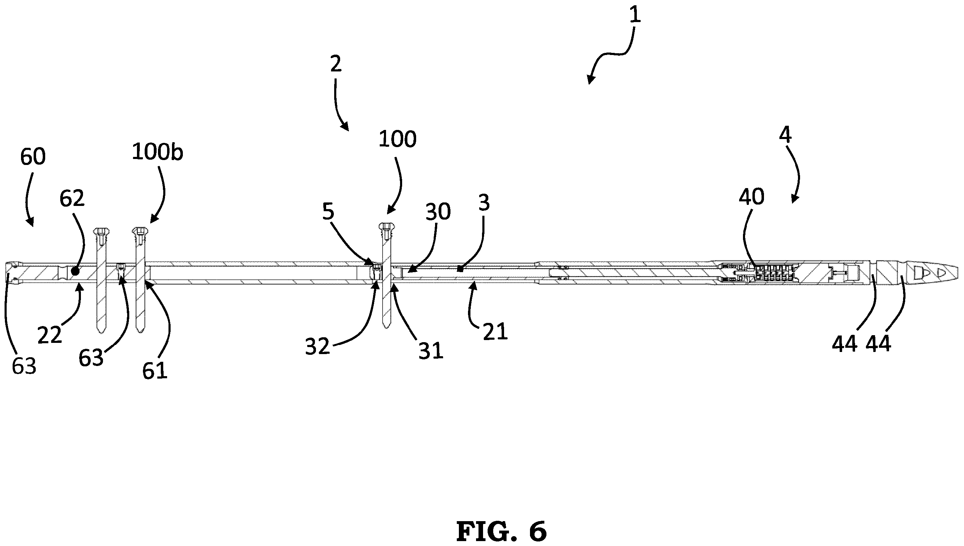

Figure 5 shows a perspective view of the intramedullary nail 1, while Figure 6 shows a sectional view of the intramedullary nail 1 (in a longer variant) with fixing screws 100, 100b which will be further described.

The first rod 3 comprises a first end 30 axially displaceable within the tube 2. Preferably, the second hole 32 is closer to the first end 30 than the first hole 31. In possible variants, the second hole 32 and the first hole 31 could be inverted in position, or even aligned at the same axial position if two separate axial slots are respectively provided.

Preferably, in a non-limiting embodiment, the first hole 31 is adjacent to the second hole 32.

Preferably, in some embodiments, the first hole 31 and the second hole 32 of the first rod 3 are configured for remaining within an outline of the first through slot 21 during the axial displacement of the latter.

Preferably, the tube 2 further comprises a distal portion 60 which comprises at least one fourth hole 61 configured to house a third fixing screw 100b.

Preferably, the distal portion 60 further comprises a second through slot 22. Preferably the second through slot 22 is longitudinally aligned along the axial direction and aligned with the first through slot 21. In an alternative, the second through slot may not be aligned to the first through slot, as far as the main axial dimension of the second slot remains aligned with the longitudinal axis of the first slot.

Preferably, the distal portion 60 further comprises a second rod 62 extending in the axial direction and telescopically coupled inside the tube 2.

Preferably, the second rod 62 is configured for axial displacement within the tube 2, and it is the second rod 62 that includes the at least one fourth hole 61; in the example there are three holes 61 (only one indicated in Figure 5 and Figure 6).

The at least one fourth hole 61 is configured for axial displacement along the second through slot 22 to displace the third fixing screw for further transporting a bone segment and lengthening of the long bone, in necessary.

Preferably, the second rod 62 is adapted to protrude with its mobile end 63 beyond an axial length of the tube 2, if necessary, during lengthening of the long bone.

In particular, during its displacement the first rod 3 does not come into contact with the second rod 62. In other words, the second rod 62 is configured for axial displacement within the tube 2 in a free manner, being constrained only by fixation screws inserted in the at least one fourth hole 61.

In a possible variant, the at least one fourth hole 62 can be slanted (or skewed) in the axial direction and is thus inclined with respect to the first hole 31.

Preferably, in the intramedullary nail 1, the holes 31 and 61 are oriented in a radial direction.

Preferably, two or more locking holes are oriented in parallel relative to one another.

Preferably, the second rod 62 comprises a second guide element 63 housed in a respective hole and axially displaceable in the axial direction.

The guide element 63 remains enclosed within an outer contour of the second through slot 22 and is configured to prevent a relative rotation of the second rod 62 with respect of the tube 2.

The guide element 63 constraints the rotation of the second rod 62 within the tube 2, so as to maintain the at least one fourth hole 61 within the profile of the second through slot, even during activation of the drive.

Advantageously, the guide element 63 remains inside the volume defined by the tube 2 of the intramedullary nail 1.

In particular, advantageously, the guide element 63 does not protrude outside of the body of the tube 2 defined by the outer contour of the second through slot 22, so that during the axial displacement the guide element 63 does not interfere with the surrounding tissue, improving tolerability of the implant.

The hole which houses the guide element 63 is preferably threaded, and the guide element 63 is preferably a guide screw comprising a threaded stem which is screwed in the respective hole.

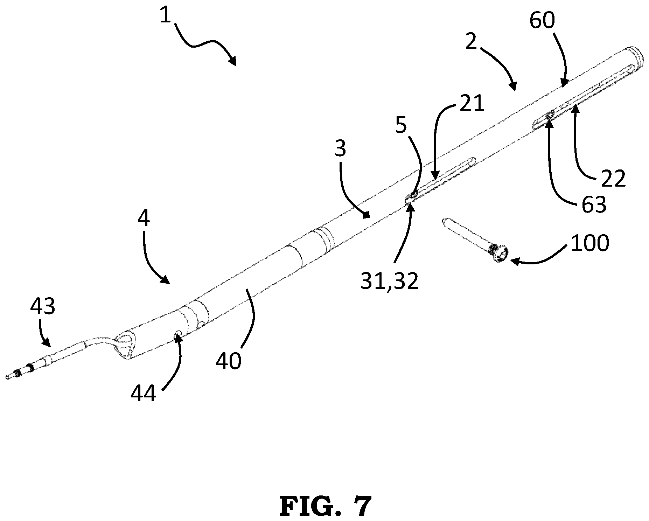

Figure 7 shows a further perspective view of the intramedullary nail 1, with a fixing screw 100.

As described above, the first rod 3 comprises a first hole 31 transversal to the axial direction and configured to house the first fixing screw 100.

The first hole 31 is configured for axial displacement along the first through slot 21 to displace the first fixing screw 100 in order to transport a bone segment and achieve the planned treatment, for example for lengthening the long bone.

The fixing screws are configured for locking the various elements of the intramedullary nail 1 in bone fragments of the long bone; in this way, the intramedullary nail can be connected to bone fragments of the long bone to be fixed in all directions and all rotational directions. The intramedullary nail 1 can thus be connected fixedly to the bone fragments in all degrees of freedom.

As already described, even in absence of the fixing screw 100, the guide element 5 remains enclosed within an outer contour of the first through slot 21 and prevents a relative rotation of the first rod 3 with respect of the tube 2, thus making it possible to provide a displacement of the rod 3, in particular a reverse displacement to 'recharge' the distraction capability. This 'recharge' feature become particularly advantageous when further lengthening of the bone is required, and the fixing screw 100 has already reached the end of the first through slot 21.

Advantageously, the guide element 5 constraints the rotation of the first rod 3 within the tube 2, so as to maintain the first hole 31 always within the profile of the first through slot 21, for further access with a fixing screw 100 at all times, even after reverse displacement.

The intramedullary nail 1 preferably further comprises a proximal portion 40.

In the preferred embodiment, the proximal portion 40 encloses the drive 4; in possible variants, the drive could be provided in a different location, for example distally.

The proximal portion 40 comprises at least one third hole 44 transversal to the axial direction. Preferably, the third hole is parallelly aligned with the first hole 31. The at least one third hole 44 is also configured to house a second fixing screw (not shown). Preferably, the third hole 44 remains fixed within a main body of the intramedullary nail 1.

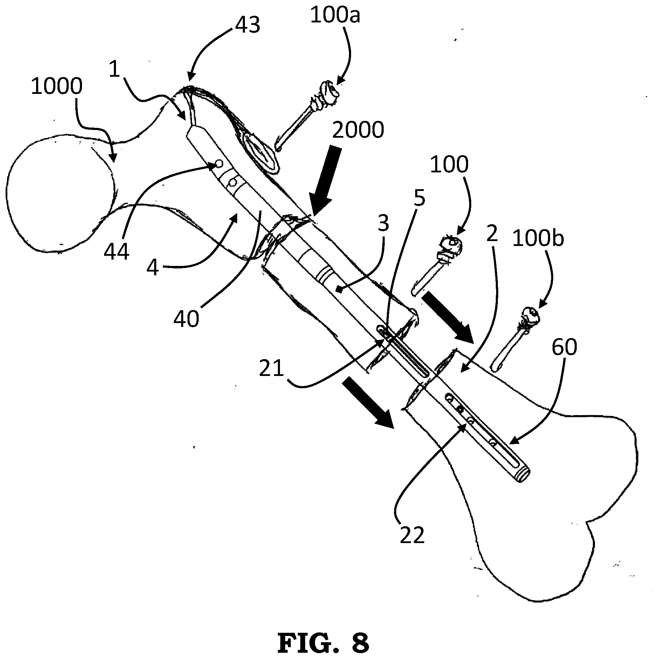

Figure 8 shows the application of the intramedullary nail 1 to a long bone 1000 having a relevant bone defect and requiring lengthening.

In this example, the length of the bone 1000 is initially maintained by stabilizing the upper and lower part of the bone to the intramedullary nail 1, by means of screws 100a and 100b.

An osteotomy 2000 is performed near the part of the missing bone where the biological vitality is greatest, thus defining a bone fragment which can be transported.

The resulting bone segment is stabilized by means of a further fixing screw or "pin" 100 at the central sliding part of the intramedullary nail 1, in connection with the first through slot 21 in which the first rod 3 is displaced.

Carried by the pin 100, the bone segment is transported axially, up to contact and abutment with the further distal bone fragment.

The intramedullary nail 1 is thus configured to transport the bone segment until it meets the distal part, as indicated by the two parallel arrows.

When the transported bone segment carried by the pin 100 contacts the distal bone fragment, the distal portion 60 of the nail 1 includes the second rod 62 already described, which allows a further displacement together with the first rod 3 and its associated first through slot 21, allowing further lengthening of the long bone 1000.

In certain cases, the stroke of displacement offered by the intramedullary nail 1 may be not sufficient, for example because it is not possible to implant a sufficiently long intramedullary nail in the long bone of the specific patient.

In this case, to achieve further displacement, the pin 100 can be removed from the bone segment, and the rod 3 and the associated first hole 31 can be retracted employing the drive.

Once the rod has been retracted to a 'recharged' position, the pin 100 (or a replacement thereof) can be inserted in a different location on the bone segment to continue displacement thereof, to achieve a further lengthening of the bone 1000.

In other words, for lengthening of bones, if the stroke provided by the first rod 3 is not sufficient, for example due to the size of the long bone or of the intramedullary nail, the transport pin 100 can be removed, the rod 3 returned to a more retracted position in a controlled manner by the presence of the guide element 5, and the lengthening can then be resumed by reinserting the pin 100, without removing the intramedullary nail 1 from the bone 1000.

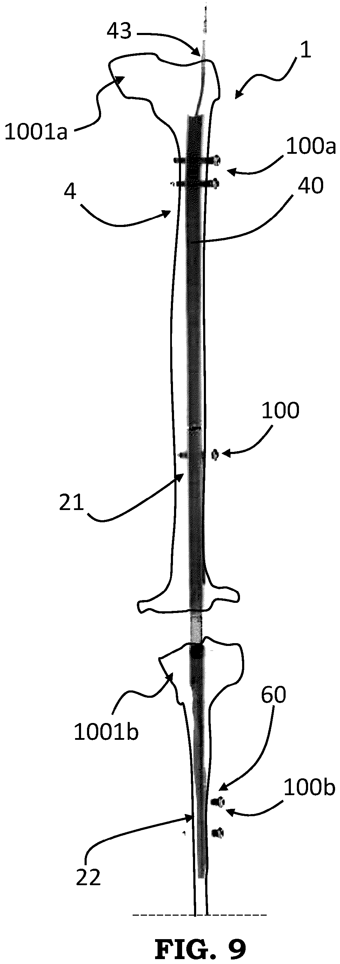

Figure 9 shows a second application of the intramedullary nail of Figure 1 across a knee joint including two long bones, respectively femur 1001a and tibia 1001b.

For knee arthrodesis applications, a distal part of a femur 1001a or the femur 1001a itself is transported to the tibial plateau of the tibia 1001b, by means of the first rod 3 displacing within the first through slot 21 and associated pin 100.

In the application, the pins 100b are engaged in the distal portion 60 in such a manner that sliding thereof in the second through slot 22 is prevented. In this manner, position of the tibia 1001b is constrained by the pins 100b and bone lengthening of the femur can be provided, for example to recover a gap generated by a removal of a knee joint prosthesis.

Considering the above-mentioned description, the person skilled in the art will be allowed to devise further modifications and variants, in order to meet contingent and specific requirements.

For example, the intramedullary nail can be varied in size, especially in its length, so it can be used for different applications.

The here-described embodiments are therefore to be considered as illustrative and non-limiting examples of the invention.

## Claims

1. An intramedullary nail (1) for a long bone, comprising:
- a tube (2) extending in an axial direction of the intramedullary nail (1), said tube (2) comprising a first through slot (21) longitudinally aligned along said axial direction;
- a first rod (3) extending in said axial direction and telescopically coupled inside said tube (2), said first rod (3) being further configured for axial displacement within said tube (2),
wherein said first rod (3) comprises a first hole (31) transversal to said axial direction and configured for housing a first fixing screw (100), said first hole (31) being configured for axial displacement along said first through slot (21) to displace said first fixing screw (100) in said axial direction;
**characterized in that** said first rod (3) further comprises:
- a second hole (32) transversal to said axial direction,
- a guide element (5) housed in said second hole (32) and axially displaceable in said axial direction, said guide element (5) being enclosed within an outer contour of said tube (2) and configured to prevent a relative rotation of said first rod (3) with respect of said tube (2).

2. The intramedullary nail (1) according to claim 1, wherein said second hole (32) is threaded, wherein said guide element (5) is a guide screw comprising a threaded stem (51) and a head (52), wherein said threaded stem (51) is screwed in said second hole (32) and wherein said head (52), during axial displacement, remains enclosed within said outer contour so as to not protrude out of said tube (2).

3. The intramedullary nail according to claim 1 or 2, wherein said first rod (3) comprises a first end (30) axially displaceable within said tube (2), said second hole (32) being closer to said first end (30) than said first hole (31), said first hole (31) being preferably adjacent to said second hole (32).

4. The intramedullary nail according to any one of claims 1 to 3, further comprising a drive (4) configured for axially displacing said first rod (3) within said tube (2).

5. The intramedullary nail according to claim 4, wherein said drive (4) comprises an electric motor (41) and a screw transmission (42), said electric motor (41) powering said screw transmission (42), said screw transmission (42) acting on said first rod (3) for a reversible displacement thereof.

6. The intramedullary nail according to claim 4 or 5, further comprising a proximal portion (40) enclosing said drive (4), wherein said proximal portion (40) further comprises at least one third hole (44) transversal to said axial direction, said at least one third hole (44) configured to house a second fixing screw.

7. The intramedullary nail according to claim 6, wherein said third hole (44) is fixed within a main body of said intramedullary nail (1).

8. The intramedullary nail according to any one of claims 4 to 7, further comprising an electric power supply device for powering said drive (4), preferably said electric power supply device comprising an induction coil.

9. The intramedullary nail according to any one of claims 1 to 8, wherein said tube (2) further comprises a distal portion (60), said distal portion (60) further comprising at least one fourth hole (61) configured to house a third fixing screw.

10. The intramedullary nail according to claim 9, wherein said distal portion (60) further comprises a second through slot (22) longitudinally aligned along said axial direction, said distal portion (60) further comprising a second rod (62) extending in said axial direction and telescopically coupled inside said tube (2), said second rod (62) being further configured for axial displacement within said tube (2), said second rod (62) including said at least one fourth hole (61), said at least one fourth hole (61) being configured for axial displacement along said second through slot (22) to further transport said third fixing screw.

11. The intramedullary nail according to claim 10, wherein said second rod (62) further comprises a second guide element (63) housed in a hole transversal to said axial direction, said second guide element (63) being axially displaceable in said axial direction and enclosed within an outer contour of said tube (2) and configured to prevent a relative rotation of said second rod (62) with respect of said tube (2).

12. The intramedullary nail according to claim 9 to 11, wherein said at least one fourth hole (61) is slanted in said axial direction and is inclined with respect to said first hole (31).

13. The intramedullary nail according to any one of claims 1 to 12, wherein said first hole (31) and said second hole (32) are configured for remaining within an outline of said first through slot (21) during said axial displacement.

14. The intramedullary nail according to any one of claims 1 to 13, wherein said tube (2) comprises a hollow shell and wherein said first through slot (21) comprises two respective cutouts (21a, 21b) in said hollow shell, said two respective cutouts (21a, 21b) being mutually transversally aligned.

15. The intramedullary nail according to any one of claims 1 to 14, wherein said guide element (5) is configured for constraining a rotation of said first rod (3) within said tube (2), so as to maintain said first hole (31) within a profile of said first through slot (21).
